# EUROPEAN PATENT SPECIFICATION

(11) **EP 1 885 309 B1**
(45) Date of publication and mention of the grant of the patent: **09.10.2013**
(21) Application number: 06726984.5
(22) Date of filing: 04.05.2006
(51) Int. Cl.: A61F 13/06

(54) **IMPROVED BUNION CUSHION**
VERBESSERTES PFLASTER FÜR SCHWEILEN
PANSEMENT AMELIORE POUR OIGNON

(30) Priority: 12.05.2005 GB 0509831
(43) Date of publication of application: 13.02.2008
(73) Proprietor: LRC Products Limited, Slough, Berkshire SL1 3UH (GB)
(72) Inventor: BAYLY, Mark, Eltham, Victoria 3095 (AU); MCCARTHY, Justin, Fairfield, Victoria 3078 (AU); RAPER, Andrew, Hampton, Victoria 3188 (AU)
(74) Representative: Carlin, Robert George
(86) International application number: PCT/GB2006/001607
(87) International publication number: WO 2006/120385

(56) References cited:
- WO-A-97/21404
- GB-A- 693 543
- GB-A- 765 434
- GB-A- 835 303
- GB-A- 981 084
- US-A- 2 585 629

## Description

The present invention relates to a bunion cushion and particularly, but not exclusively, to a bunion cushion for locating about a toe of a user.

It is well known to use a padded cushion in order to protect a bunion and thereby alleviate pain caused by the bunion. However, conventional bunion cushions tend to be held in place adjacent a bunion in a way which is often uncomfortable for the user. Furthermore, despite the use of ventilating holes in the surface of some prior art bunion cushions, it is frequently the case that a user will perspire in the area of a bunion cushion and this results in the need to frequently replace or clean a bunion cushion in order to maintain an acceptable level of hygiene. In addition, conventional bunion cushions are generally unsightly and this can cause embarrassment to the user.

It is an object of the present invention to provide a bunion cushion which is comfortable to wear, has an effective means for being secured in a desired position, has an improved means for providing ventilation, and/or an aesthetically pleasing appearance.

A first aspect of the present invention provides a bunion cushion comprising securing means for securing the bunion cushion adjacent the skin of a user, wherein a surface extends over the securing means and has one or more protrusions extending therefrom, and wherein said surface, in use, is placed adj acent the skin of a user of the bunion cushion.

At least one protrusion may be located adjacent an edge of said surface so that, in use, said edge is spaced from the skin of a user by the at least one protrusion. A region of said surface extending from adjacent one edge portion of said surface to adjacent another edge portion of said surface may be provided with at least one protrusion extending therefrom so that, in use, said region of said surface is spaced from the skin of a user so as to provide fluid communication between said two edge portions.

The surface on the securing means may have a closed loop shape. The or each protrusion on the securing means may be provided as a ridge. These ridges may extend radially in line with a centre or the closed loop shape of the securing means. At least one of the ridges may have a semi-circular cross-section.

The bunion cushion may further comprise a cushion portion for locating adjacent a bunion, wherein said surface extends over the cushion portion. Said surface on the bunion cushion may have a closed loop shape. Furthermore, the or each protrusion on the cushion portion may be provided as a ridge. Ideally, the or each ridge extends radially in line with a centre of the closed loop shape. At least one ridge may have a semi-circular cross-section.

Furthermore, the or each protrusion may have a free end located in a plane perpendicularly spaced from said surface by a distance of approximately 0.5 mm. Also, any two adjacent protrusions are ideally spaced from one another by approximately 2 mm.

The securing means may comprise a closed loop of material wherein a lengthwise portion of said closed loop of material is planar. A second lengthwise portion may be provided in said closed loop of material, extending at an angle to the plane of said first lengthwise portion.

Ideally, said angle is approximately 90°. Alternatively, said angle is an acute angle. The angle may, for example, have a value from 30° to 45° and is preferably 45°. Furthermore, said angle is preferably formed between surfaces of the securing means which, in use, are placed adjacent the skin of a user of the bunion cushion.

Ideally, the first and second lengthwise portions are arranged so as to define a generally triangular shape. The first lengthwise portion may comprise two generally straight elements arranged at an angle to one another. The second lengthwise portion and the two elements of the first lengthwise portion may be connected to one another by three curved portions so as to define a closed loop of material having a generally triangular shape.

The first lengthwise portion may adjoin the cushion portion by means of one of said curved portions. Ideally, the first lengthwise portion is located in the same plane as the cushion portion.

A third aspect of the present invention provides a bunion cushion manufactured from a clear material. Ideally, the bunion cushion comprises a cushion portion for locating adjacent a bunion and securing means for securing the cushion portion adjacent the skin of a user. The material of the bunion cushion ideally exhibits a Shore 00 hardness of from 30 to 65 and preferably of 55. Also, the material of the bunion cushion preferably has a low tensile strength. For example, the tensile strength (ult.) may be from 1 to 10 MPa, preferably from 2 to 5 MPa, and is ideally 2.8 MPa (400 psi). Preferably, the bunion cushion is manufactured from Monoprene® TPE MP-1798 thermoplastic elastomer.

A bunion cushion may also be provided in which either of the first, second or third aspects of the present invention combine with one or more of the other aspects of the present invention.

An embodiment of the present invention will now be described with reference to the accompanying drawings, in which:
Figure 1 is a top view of a bunion cushion according to the present invention;
Figure 2 is a front view of the bunion cushion shown in Figure 1;
Figure 3 is a top view of the bunion cushion shown in Figure 1 wherein lines are provided showing hidden detail;
Figure 4 is a front view of the bunion cushion shown in Figure 1 wherein lines are provided showing hidden detail;
Figure 5 is a back view of the bunion cushion shown in Figure 1 wherein lines are provided showing hidden detail;
Figure 6 is a bottom view of the bunion cushion shown in Figure 1 wherein lines are provided showing hidden detail;
Figure 7 is a right-hand side view of the bunion cushion shown in Figure 1 wherein lines are provided showing hidden detail; and
Figure. 8 is a left-hand view of the bunion cushion shown in Figure 1 wherein lines are provided showing hidden details.

A bunion cushion 2 according to the present invention is shown in the accompanying drawings as comprising a cushion portion 4 and means 6 for securing the cushion portion in position over a bunion.

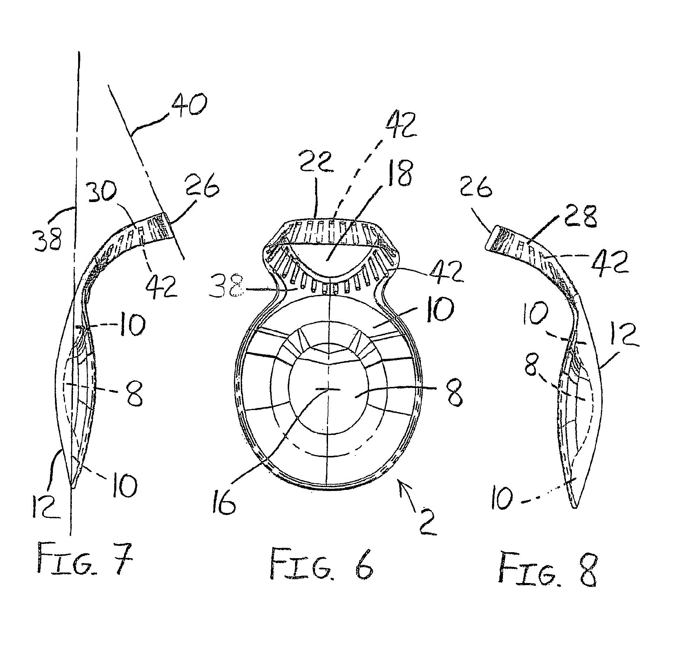

The cushion portion 4 has a generally dish-like shape so as to provide a concave central area 8 surrounded by a perimeter ring 10. The concave central area 8 and the perimeter ring 10 are most clearly shown in the bottom view of Figure 6 and the side views of Figures 7 and 8 of the accompanying drawings.

It will be understood that the perimeter ring 10 functions in use to provide circumferential support about a bunion to be protected by the bunion cushion 2. The inner diameter of the perimeter ring 10 is therefore sufficiently large to remain radially spaced from a bunion to be protected. The radial depth of the perimeter ring 10 (in other words, the distance between the inner and outer diameters of the perimeter ring 10) is such as to provide a comfortable and cushioning contact area between the bunion cushion 2 and the user. Similarly, the lateral depth of the perimeter ring 10 (in other words, the thickness of the perimeter ring 10) is sufficient to provide a comfortable and cushioning affect.

The outer surface 12 of the cushion portion 4 has a convex shape with an apex 14 located over the centre 16 of the perimeter ring 10. The use of a convex shape for the outer surface 12 assists in the provision of an interior concave shape for the central area 8 and ensures the cushion portion 4 can, in use, remain spaced from a bunion being protected by the bunion cushion 2.

It will be appreciated by the skilled person that the size and shape of the cushion portion 4 may differ to that shown in the accompanying drawings. The perimeter ring 10 should be sufficiently large to extend around a bunion to be protected and the central area 8 should have a sufficiently curved interior concave surface and/or be sufficiently raised by the perimeter ring 10 to be capable of extending over a bunion without making contact therewith. In this way, the cushion portion 4 will provide protection for a bunion without irritating the bunion and causing pain to the user.

The cushion portion 4 is retained in position over a bunion by means of securing means 6 attached to the cushion portion 4. The securing means 6 comprises a closed loop of material forming an aperture 18 through which, in use, a toe of the user is extended. The loop of material is shaped and positioned so as to give a comfortable fit around a toe and so as to allow the bunion cushion 2, and particularly the cushion portion 4, to lie flat more readily along the edge of a foot where the bunion is located.

In order to assist in the securing means 6 itself lying flat about a toe of a user, the loop of material is formed from a generally flat strip of material which is bent, folded, twisted or otherwise shaped along its length so as to lie flat against the skin as it extends about a toe. The outer surface 20 of the loop may therefore have, for example, a generally frustro-conical shape. With such an arrangement, a first edge 22 of the strip of material defines a smaller opening than a second edge 24 of the material. As will be evident from the accompanying drawings, the second edge 24 is located adjacent the cushion portion 4 with the first edge 22 located remote from the cushion portion 4. A toe may then be inserted past the second edge 24 so as to exit the aperture 18 via the first edge 22. In other words, in use, the second edge 24 is positioned nearer to the joint between the toe and the foot of the user. In this way, the tapering frustro-conical shape of the loop ensures the securing means is able to extend about the toe joint and lay flat against the skin. As a result, the loop (and the bunion cushion 2 as a whole) is comfortable for the user to wear.

It will be appreciated that the strip of material forming the securing means 6 may take a form different to that referred to above. For example, whilst the half of the loop remote from the cushion portion 4 may take a part frustro-conical shape, the half of the loop adjacent to the cushion portion 4 may be flat and planar, or become progressively more flat and planar towards the cushion portion 4. Alternatively, the loop may comprise a number of planar lengths arranged at angles to one another. A faceted closed loop of material may be thereby provided.

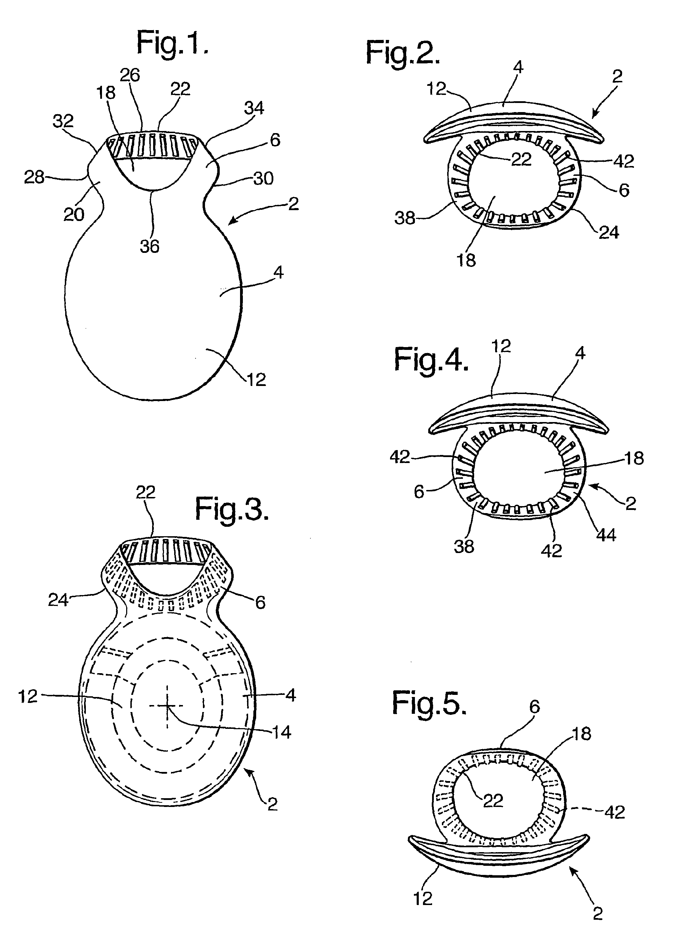

Preferentially, the aperture 18 approximates a triangular shape defined by three approximately straight portions 26, 28, 30 of the loop. The straight portions may be interconnected by curved portions with the securing means 6 adjoining the cushion portion 4 by means of one of the curved portions. With reference to Figure 1, it will be seen that the securing means 6 of the bunion cushion 2 comprises three generally straight portions 26, 28, 30 connected by three curved portions 32, 34, 36. A generally triangular-shaped aperture 18 is thereby provided. Although not shown in the accompanying drawings, the resiliently flexible material of the securing means 6 allows two of the straight portions 28, 30 to bend relative to the cushion portion 4 and lie, in a relaxed and unstretched state, in the same plane 38 as the cushion portion 4. These two straight portions 28, 30 are connected to one another and to the cushion portion 4 by means of one of the curved portions 36. The other of the three generally straight portion 26 joins the first and second generally straight portions 28, 30 by means of two curved portions 32, 34 and lies in a plane 40 arranged at an angle to the plane 38 of said first and second portions 28, 30.

The aforementioned two planes in which the straight portions 26, 28, 30 lie may extend perpendicularly relative to one another as in the embodiment of the accompanying drawings. Alternatively, the two planes of the generally straight portions 26, 28, 30 may be arranged such that the portions of one plane are located at an acute angle relative to the portion of the other plane, for example, at 30° or 45°.

It will be understood that modifications may be made to the securing means 6 shown in the accompanying drawings. For example, one or more of the generally straight portions 26, 28, 30 may be formed to curve out of its respective plane 38, 40 when in a relaxed and unstretched state. Also, one or more of the straight portions may alternatively or additionally be twisted along its length.

The bunion cushion 2 is further provided with means for raising the securing means 6 and/or cushion portion 4 from the skin of a user so as to allow a circulation of air between the skin and the securing means and cushion portion. The bunion cushion 2 shown in the accompanying drawings has a plurality of ridges 42 which assist in ensuring a circulation of air between the securing means 6 and the toe of a user. The ridges 42 are each of an elongate semi-cylindrical shape (i.e. of a semi-circular cross-section) and are formed integrally with the inner surface 44 of the aforementioned loop material (i.e. the surface of said loop which, in use, locates adjacent the toe). The ridges 42 are straight and extend radially from an approximate central location of the aperture 18. Furthermore, the ridges are approximately 1 mm wide and extend approximately 0.5 mm from the inner surface 44 of the loop. The lengths of the ridges also vary depending upon the location along the length of the loop (see Figure 6). For example, a ridge 42 arranged at an angle to a generally straight portion 26, 28, 30 has a longer length than a ridge 42 extending directly across (i.e. perpendicularly to) a straight portion 26, 28, 30. It will be seen from the accompanying drawings that a ridge 42 extends from adjacent the first edge 22 to a position adjacent the second edge 4 of the loop so as to ensure that the full width of the loop is raised from the skin of the user. In addition, in order to provide sufficient airflow between the ridges 42, adjacent ridges 42 are spaced by approximately 2 mm.

The ridges 42 located on a portion of securing means 6 adjoining the cushion portion 4 may extend from the securing means 6 across the cushion portion 4 (although this arrangement is not shown in the accompanying drawings). Indeed, the perimeter ring 10 of the cushion portion 4 may be provided with ridges or other means similar or identical to that used on the securing means 6 for providing air circulation. For example, ridges may radially extend so as to be generally in line with the centre of the concave central area 8. In this way, air may flow, in use, from the exterior of the bunion cushion 2 into the region between the perimeter ring 10 and the skin of a user, and then into the concave central area 8. A cooling airflow is thereby provided which may circulate about a bunion being protected by the bunion cushion 2. This has the affect of keeping the skin of the user at a comfortable temperature and reduces localised perspiration which, in turn, reduces the frequency with which the bunion cushion 2 must be replaced or cleaned in order to maintain an acceptable level of hygiene.

## Claims

1. A bunion cushion (2) comprising securing means (6) for securing the bunion cushion (2) adjacent the skin of a user, wherein a surface (44) extends over the securing means (6) and has one or more protrusions (42) extending therefrom, and wherein said surface (44), in use, is placed adjacent the skin of a user of the bunion cushion (2).

2. A bunion cushion (2) as claimed in claim 1, wherein at least one protrusion (42) is located adjacent an edge (22) of said surface so that, in use, said edge (22) is spaced from the skin of a user by the at least one protrusion (42).

3. A bunion cushion (2) as claimed in claim 1 or 2, wherein a region of said surface (44) extending from adjacent one edge portion (22) of said surface (44) to adjacent another edge portion (24) of said surface is provided with at least one protrusion (42) extending therefrom so that, in use, said region of said surface (44) is spaced from the skin of a user so as to provide fluid communication between said two edge portions (22.24).

4. A bunion cushion (2) as claimed in any of claims 1 to 3, wherein said surface (44) on the securing means (6) has a closed loop shape.

5. A bunion cushion (2) as claimed in any of claims 1 to 3, wherein the or each protrusion (42) on the securing means (6) is provided as a ridge.

6. A bunion cushion (2) as claimed in claim 4, wherein the or each protrusion (42) on the securing means (6) is provided as a ridge.

7. A bunion cushion (2) as claimed in any of the preceding claims, the bunion cushion (2) further comprising a cushion portion (4) for locating adjacent a bunion, wherein said surface (44) extends over the cushion portion (4).

8. A bunion cushion (2) as claimed in claim 7, wherein said surface (44) on the bunion cushion (2) has a closed loop shape.

9. A bunion cushion (2) as claimed in claim 7, wherein the or each protrusion (42) on the cushion portion (4) is provided as a ridge.

10. A bunion cushion (2) as claimed in claim 8, wherein the or each protrusion (42) on the cushion portion (4) is provided as a ridge.

11. A bunion cushion (2) as claimed in claim 6 or 10, wherein the or each ridge (42) extends radially in line with a centre of the closed loop shape.

12. A bunion cushion (2) as claimed in any of claims 5, 6 and 9 to 11, wherein at least one ridge (42) has a semi-circular cross-section.

13. A bunion cushion (2) as claimed in any of the preceding claims, wherein the or each protrusion (42) has a free end located in a plane perpendicularly spaced from said surface (44) by a distance of approximately 0.5 mm.

14. A bunion cushion (2) as claimed in any of the preceding claims, wherein any two adjacent protrusions (42) are spaced from one another by approximately 2 mm.

15. A bunion cushion (2) as claimed in any of the preceding claims, the securing means (6) comprising a closed loop of material, wherein a lengthwise portion (26) of said closed loop of material is planar.

16. A bunion cushion (2) as claimed in claim 15, wherein said closed loop of material comprises a second lengthwise portion (28,30) which extends at an angle to the plane of said first lengthwise portion (26).

17. A bunion cushion (2) as claimed in claim 16, wherein said angle is approximately 90°.

18. A bunion cushion (2) as claimed in claim 16, wherein said angle is an acute angle.

19. A bunion cushion (2) as claimed in claim 18, wherein said angle is from 30° to 45° and is preferably 45°.

20. A bunion cushion (2) as claimed in any of claims 16 to 19, wherein said angle is formed between surfaces of the securing means (6) which, in use, are placed adjacent the skin of a user of the bunion cushion (2).

21. A bunion cushion (2) as claimed in any of claims 16 to 20, wherein the first and second lengthwise portions (26,28,30) are arranged so as to define a generally triangular shape.

22. A bunion cushion (2) as claimed in claim 21, wherein the first lengthwise portion comprises two generally straight elements (28,30) arranged at an angle to one another.

23. A bunion cushion (2) as claimed in claim 22, wherein the second lengthwise portion (26) and the two elements (28,30) of the first lengthwise portion are connected to one another by three curved portions (32,34,36) so as to define a closed loop of material having a generally triangular shape.

24. A bunion cushion (2) as claimed in claim 23, wherein the first lengthwise portion (28,30) adjoins the cushion portion (4) by means of one of said curved portions (36).

25. A bunion cushion (2) as claimed in claim 24, wherein the first lengthwise portion (28,30) is located in the same plane as the cushion portion (4).

26. A bunion cushion (2) as claimed in claim 24, wherein the first lengthwise portion (28,30) is located in a plane oriented at an angle to a plane in which the cushion portion (4) is located.

27. A bunion cushion (2) as claimed in any of the preceding claims, manufactured from a clear material.

28. A bunion cushion (2) as claimed in claim 27, wherein the material of the bunion cushion (2) exhibits a Shore 00 hardness of from 30 to 65.

29. A bunion cushion (2) as claimed in claim 28, wherein the material of the bunion cushion (2) exhibits a Shore 00 hardness of 55.

30. A bunion cushion (2) as claimed in any of claims 27 to 29, wherein the material of the bunion cushion (2) has a low tensile strength.

31. A bunion cushion (2) as claimed in claim 30, wherein the tensile strength (ult.) is from 1 to 10 MPa.

32. A bunion cushion (2) as claimed in claim 31, wherein the tensile strength is from 2 to 5 MPa.

33. A bunion cushion (2) as claimed in claim 32, wherein the tensile strength is 2.8 MPa (400 psi).

34. A bunion cushion (2) as claimed in any of claims 27 to 33, wherein the bunion cushion (2) is manufactured from TPE MP-1798 thermoplastic elastomer.

## Patentansprüche

1. Ballenpflaster (2), umfassend ein Befestigungsmittel (6) zur Befestigung des Ballenpflasters (2) nahe der Haut eines Benutzers, wobei sich eine Fläche (44) über das Befestigungsmittel (6) erstreckt und einen oder mehrere Vorsprünge (42) hat, die sich davon erstrecken, und wobei die Fläche (44) im Gebrauch nahe der Haut eines Benutzers des Ballenpflasters (2) platziert ist.

2. Ballenpflaster (2) nach Anspruch 1, wobei mindestens ein Vorsprung (42) in der Nähe eines Rands (22) der Fläche angeordnet ist, so dass der Rand (22) im Gebrauch von der Haut eines Benutzers durch den mindestens einen Vorsprung (42) beabstandet ist.

3. Ballenpflaster (2) nach Anspruch 1 oder 2, wobei ein Bereich der Fläche (44), der sich von nahe einem Randabschnitt (22) der Fläche (44) bis zu nahe einem anderen Randabschnitt (24) der Fläche erstreckt, mit mindestens einem sich davon erstreckenden Vorsprung (42) versehen ist, so dass der Bereich der Fläche (44) im Gebrauch von der Haut eines Benutzers beabstandet ist, um für eine Fluidverbindung zwischen den beiden Randabschnitten (22, 24) zu sorgen.

4. Ballenpflaster (2) nach einem der Ansprüche 1 bis 3, wobei die Fläche (44) an dem Befestigungsmittel (6) die Form einer geschlossenen Schlaufe hat.

5. Ballenpflaster (2) nach einem der Ansprüche 1 bis 3, wobei der oder jeder Vorsprung (42) am Befestigungsmittel (6) als ein Grat vorgesehen ist.

6. Ballenpflaster (2) nach Anspruch 4, wobei der oder jeder Vorsprung (42) am Befestigungsmittel (6) als ein Grat vorgesehen ist.

7. Ballenpflaster (2) nach einem der vorhergehenden Ansprüche, wobei das Ballenpflaster (2) ferner einen Polsterabschnitt (4) zur Anordnung in der Nähe eines Ballens umfasst, wobei sich die Fläche (44) über den Polsterabschnitt (4) erstreckt.

8. Ballenpflaster (2) nach Anspruch 7, wobei die Fläche (44) an dem Ballenpflaster (2) die Form einer geschlossenen Schlaufe hat.

9. Ballenpflaster (2) nach Anspruch 7, wobei der oder jeder Vorsprung (42) am Polsterabschnitt (4) als ein Grat vorgesehen ist.

10. Ballenpflaster (2) nach Anspruch 8, wobei der oder jeder Vorsprung (42) am Polsterabschnitt (4) als ein Grat vorgesehen ist.

11. Ballenpflaster (2) nach Anspruch 6 oder 10, wobei sich der oder jeder Grat (42) radial auf eine Mitte der geschlossenen Schlaufenform ausgerichtet erstreckt.

12. Ballenpflaster (2) nach einem der Ansprüche 5, 6 und 9 bis 11, wobei mindestens ein Grat (42) einen halbkreisförmigen Querschnitt hat.

13. Ballenpflaster (2) nach einem der vorhergehenden Ansprüche, wobei der oder jeder Vorsprung (42) ein freies Ende hat, das in einer Ebene liegt, die mit einem Abstand von ungefähr 0,5 mm senkrecht von der Fläche (44) beabstandet ist.

14. Ballenpflaster (2) nach einem der vorhergehenden Ansprüche, wobei jeweils zwei beliebige benachbarte Vorsprünge (42) ungefähr 2 mm voneinander beabstandet sind.

15. Ballenpflaster (2) nach einem der vorhergehenden Ansprüche, wobei das Befestigungsmittel (6) eine geschlossene Materialschlaufe umfasst, wobei ein Längsabschnitt (26) der geschlossenen Materialschlaufe planar ist.

16. Ballenpflaster (2) nach Anspruch 15, wobei die geschlossene Materialschlaufe einen zweiten Längsabschnitt (28, 30) umfasst, der sich in einem Winkel zu der Ebene des ersten Längsabschnitts (26) erstreckt.

17. Ballenpflaster (2) nach Anspruch 16, wobei der Winkel ungefähr 90° beträgt.

18. Ballenpflaster (2) nach Anspruch 16, wobei der Winkel ein spitzer Winkel ist.

19. Ballenpflaster (2) nach Anspruch 18, wobei der Winkel zwischen 30° und 45° und vorzugsweise 45° beträgt.

20. Ballenpflaster (2) nach einem der Ansprüche 16 bis 19, wobei der Winkel zwischen Flächen des Befestigungsmittels (6) gebildet ist, die im Gebrauch nahe der Haut eines Benutzers des Ballenpflasters (2) platziert sind.

21. Ballenpflaster (2) nach einem der Ansprüche 16 bis 20, wobei der erste und der zweite Längsabschnitt (26, 28, 30) so angeordnet sind, dass sie eine allgemein dreieckige Form definieren.

22. Ballenpflaster (2) nach Anspruch 21, wobei der erste Längsabschnitt zwei allgemein gerade Elemente (28, 30) umfasst, die in einem Winkel zueinander angeordnet sind.

23. Ballenpflaster (2) nach Anspruch 22, wobei der zweite Längsabschnitt (26) und die beiden Elemente (28, 30) des ersten Längsabschnitts über drei gebogene Abschnitte (32, 34, 36) miteinander verbunden sind, um eine geschlossene Materialschlaufe mit einer allgemein dreieckigen Form zu definieren.

24. Ballenpflaster (2) nach Anspruch 23, wobei der erste Längsabschnitt (28, 30) über einen der gebogenen Abschnitte (36) an den Polsterabschnitt (4) anschließt.

25. Ballenpflaster (2) nach Anspruch 24, wobei der erste Längsabschnitt (28, 30) in derselben Ebene wie der Polsterabschnitt (4) angeordnet ist.

26. Ballenpflaster (2) nach Anspruch 24, wobei der erste Längsabschnitt (28, 30) in einer Ebene angeordnet ist, die in einem Winkel zu einer Ebene ausgerichtet ist, in der der Polsterabschnitt (4) angeordnet ist.

27. Ballenpflaster (2) nach einem der vorhergehenden Ansprüche, das aus einem klaren Material hergestellt ist.

28. Ballenpflaster (2) nach Anspruch 27, wobei das Material des Ballenpflasters (2) eine Shore 00-Härte von 30 bis 65 aufweist.

29. Ballenpflaster (2) nach Anspruch 28, wobei das Material des Ballenpflasters (2) eine Shore 00-Härte von 55 aufweist.

30. Ballenpflaster (2) nach einem der Ansprüche 27 bis 29, wobei das Material des Ballenpflasters (2) eine geringe Zugfestigkeit hat.

31. Ballenpflaster (2) nach Anspruch 30, wobei die Zugfestigkeit zwischen 1 und 10 MPa beträgt.

32. Ballenpflaster (2) nach Anspruch 31, wobei die Zugfestigkeit zwischen 2 und 5 MPa beträgt.

33. Ballenpflaster (2) nach Anspruch 32, wobei die Zugfestigkeit 2,8 MPa (400 psi) beträgt.

34. Ballenpflaster (2) nach einem der Ansprüche 27 bis 33, wobei das Ballenpflaster (2) aus dem thermoplastischen Elastomer TPE MP-1798 hergestellt ist.

## Revendications

1. Coussin pour oignon (2) comprenant un moyen de fixation (6) pour fixer le coussin pour oignon (2) en position adjacente à la peau d'un utilisateur, une surface (44) s'étendant par-dessus le moyen de fixation (6) et présentant une ou plusieurs saillies (42) s'étendant depuis celles-ci, et ladite surface (44), pendant l'utilisation, étant placée en position adjacente à la peau d'un utilisateur du coussin pour oignon (2).

2. Coussin pour oignon (2) selon la revendication 1, dans lequel au moins une saillie (42) est située en position adjacente à un bord (22) de ladite surface de telle sorte que, pendant l'utilisation, ledit bord (22) soit espacé de la peau d'un utilisateur de la distance de l'au moins une saillie (42).

3. Coussin pour oignon (2) selon la revendication 1 ou 2, dans lequel une région de ladite surface (44) s'étendant depuis une position adjacente à une portion de bord (22) de ladite surface (44) jusqu'à une position adjacente à une autre portion de bord (24) de ladite surface est munie d'au moins une saillie (42) s'étendant depuis celle-ci de telle sorte que, pendant l'utilisation, ladite région de ladite surface (44) soit espacée de la peau d'un utilisateur de manière à assurer une communication fluidique entre lesdites deux portions de bord (22, 24).

4. Coussin pour oignon (2) selon l'une quelconque des revendications 1 à 3, dans lequel ladite surface (44) sur le moyen de fixation (6) présente une forme en boucle fermée.

5. Coussin pour oignon (2) selon l'une quelconque des revendications 1 à 3, dans lequel la ou chaque saillie (42) sur le moyen de fixation (6) est prévue sous forme de crête.

6. Coussin pour oignon (2) selon la revendication 4, dans lequel la ou chaque saillie (42) sur le moyen de fixation (6) est prévue sous forme de crête.

7. Coussin pour oignon (2) selon l'une quelconque des revendications précédentes, le coussin pour oignon (2) comprenant en outre une portion de coussin (4) destinée à être positionnée de manière adjacente à un oignon, ladite surface (44) s'étendant par-dessus la portion de coussin (4).

8. Coussin pour oignon (2) selon la revendication 7, dans lequel ladite surface (44) sur le coussin pour oignon (2) présente une forme en boucle fermée.

9. Coussin pour oignon (2) selon la revendication 7, dans lequel la ou chaque saillie (42) sur la portion de coussin (4) est prévue sous forme de crête.

10. Coussin pour oignon (2) selon la revendication 8, dans lequel la ou chaque saillie (42) sur la portion de coussin (4) est prévue sous forme de crête.

11. Coussin pour oignon (2) selon la revendication 6 ou 10, dans lequel la ou chaque crête (42) s'étend radialement de manière à être alignée avec un centre de la forme en boucle fermée.

12. Coussin pour oignon (2) selon l'une quelconque des revendications 5, 6 et 9 à 11, dans lequel au moins une crête (42) présente une section transversale semi-circulaire.

13. Coussin pour oignon (2) selon l'une quelconque des revendications précédentes, dans lequel la ou chaque saillie (42) présente une extrémité libre située dans un plan espacé perpendiculairement par rapport à ladite surface (44) d'une distance d'environ 0,5 mm.

14. Coussin pour oignon (2) selon l'une quelconque des revendications précédentes, dans lequel deux saillies adjacentes quelconques (42) sont espacées l'une de l'autre d'environ 2 mm.

15. Coussin pour oignon (2) selon l'une quelconque des revendications précédentes, le moyen de fixation (6) comprenant une boucle fermée de matériau, une portion longitudinale (26) de ladite boucle fermée de matériau étant plane.

16. Coussin pour oignon (2) selon la revendication 15, dans lequel ladite boucle fermée de matériau comprend une deuxième portion longitudinale (28, 30) qui s'étend suivant un certain angle par rapport au plan de ladite première portion longitudinale (26).

17. Coussin pour oignon (2) selon la revendication 16, dans lequel ledit angle mesure environ 90°.

18. Coussin pour oignon (2) selon la revendication 16, dans lequel ledit angle est un angle aigu.

19. Coussin pour oignon (2) selon la revendication 18, dans lequel ledit angle mesure entre 30° et 45°, et de préférence vaut 45°.

20. Coussin pour oignon (2) selon l'une quelconque des revendications 16 à 19, dans lequel ledit angle est formé entre des surfaces du moyen de fixation (6) qui, pendant l'utilisation, sont placées en position adjacente à la peau d'un utilisateur du coussin pour oignon (2).

21. Coussin pour oignon (2) selon l'une quelconque des revendications 16 à 20, dans lequel lesdites première et deuxième portions longitudinales (26, 28, 30) sont disposées de manière à définir une forme généralement triangulaire.

22. Coussin pour oignon (2) selon la revendication 21, dans lequel la première portion longitudinale comprend deux éléments généralement droits (28, 30) disposés suivant un certain angle l'un par rapport à l'autre.

23. Coussin pour oignon (2) selon la revendication 22, dans lequel la deuxième portion longitudinale (26) et les deux éléments (28, 30) de la première portion longitudinale sont connectés les uns aux autres par trois portions courbes (32, 34, 36) de manière à définir une boucle fermée de matériau ayant une forme généralement triangulaire.

24. Coussin pour oignon (2) selon la revendication 23, dans lequel la première portion longitudinale (28, 30) est adjacente à la portion de coussin (4) au moyen de l'une desdites portions courbes (36).

25. Coussin pour oignon (2) selon la revendication 24, dans lequel la première portion longitudinale (28, 30) est située dans le même plan que la portion de coussin (4).

26. Coussin pour oignon (2) selon la revendication 24, dans lequel la première portion longitudinale (28, 30) est située dans un plan orienté suivant un certain angle par rapport à un plan dans lequel est située la portion de coussin (4).

27. Coussin pour oignon (2) selon l'une quelconque des revendications précédentes, fabriqué en un matériau transparent.

28. Coussin pour oignon (2) selon la revendication 27, dans lequel le matériau du coussin pour oignon (2) présente une dureté Shore 00 comprise entre 30 et 65.

29. Coussin pour oignon (2) selon la revendication 28, dans lequel le matériau du coussin pour oignon (2) présente une dureté Shore 00 de 55.

30. Coussin pour oignon (2) selon l'une quelconque des revendications 27 à 29, dans lequel le matériau du coussin pour oignon (2) présente une faible résistance à la traction.

31. Coussin pour oignon (2) selon la revendication 30, dans lequel la résistance à la traction (ult.) est comprise entre 1 et 10 MPa.

32. Coussin pour oignon (2) selon la revendication 31, dans lequel la résistance à la traction est comprise entre 2 et 5 MPa.

33. Coussin pour oignon (2) selon la revendication 32, dans lequel la résistance à la traction est de 2,8 MPa (400 psi).

34. Coussin pour oignon (2) selon l'une quelconque des revendications 27 à 33, dans lequel le coussin pour oignon (2) est fabriqué à partir d'un élastomère thermoplastique de type TPE MP-1798.
